# EUROPEAN PATENT SPECIFICATION

(11) **EP 1 592 479 B1**
(45) Date of publication and mention of the grant of the patent: **29.07.2009**
(21) Application number: 04705987.8
(22) Date of filing: 28.01.2004
(51) Int. Cl.: A61N 1/05

(54) **TACHY LEAD SYSTEM FOR SEPTAL PLACEMENT**
TACHY-LEITUNGSSYSTEM FÜR DIE SEPTUMPLATZIERUNG
SYSTEME DE DERIVATION DE TACHYCARDIE EN VUE D'UNE DISPOSITION SEPTALE

(30) Priority: 28.01.2003 US 352546
(43) Date of publication of application: 09.11.2005
(73) Proprietor: CARDIAC PACEMAKERS, INC., St. Paul, Minnesota 55112 (US)
(72) Inventor: ZHANG, Yongxing, Little Canada, Minnesota 55117 (US); BODNER, Jeffrey, P., St Paul, Minnesota 55117 (US)
(74) Representative: Joly, Jean-Jacques
(86) International application number: PCT/US2004/002220
(87) International publication number: WO 2004/067089

(56) References cited:
- US-A- 5 014 696
- US-A- 5 176 137
- US-A- 5 447 519
- US-A- 5 871 530
- US-B1- 6 178 355

## Description

### Technical Field

The present invention relates generally to a lead for stimulating or monitoring tissue. More particularly, it pertains to a lead that provides ventricular pacing and/or sensing.

### Background

Tachyarrhythmias are abnormal heart rhythms characterized by a rapid heart rate. Examples of tachyarrhythmias include supraventricular tachycardias (SVT's) such as sinus tachycardia, atrial tachycardia, and atrial fibrillation. The most dangerous tachyarrhythmias, however, are ventricular tachycardia (VT) and ventricular fibrillation (VF). Ventricular rhythms typically result in rapid and irregular contraction of the ventricles out of electromechanical synchrony with the atria.

Cardioversion and defibrillation can be used to terminate most tachyarrhythmias, including SVT's, VT, and VF. A class of cardiac rhythm management devices known as an implantable cardioverter/defibrillator (ICD) provides therapy by delivering a shock pulse to the heart, such as when the device detects fibrillation.

Another type of electrical therapy for tachycardia is antitachycardia pacing (ATP). In ATP, the heart is paced using one or more pacing pulses in an effort to correct the tachycardia. Modem ICD's typically have ATP capability so that ATP therapy is delivered to the heart when a tachycardia is detected, while a shock pulse is delivered when fibrillation occurs.

Although cardioversion/defibrillation will terminate tachycardia, it consumes a large amount of stored power from the battery and results in patient discomfort owing to the high voltage of the shock pulses. Therefore, it is desirable for the ICD to use ATP to terminate a tachyarrhythmia whenever possible.

ICD's typically include one or more leads that are implanted in or about the heart to reverse certain life threatening arrhythmias, or to stimulate contraction of the heart. Electrical energy is applied to the heart via electrodes on the leads to return the heart to normal rhythm.

A typical cardiac pacing lead is positioned within one or more chambers of the heart such that the distal end of the lead is positioned in the ventricle or atrium through a subclavian vein, while the proximal end is attached to a pacemaker which is implanted subcutaneously. Some leads also include electrodes that operate as sensors to monitor the atrium and/or ventricle of the heart, and in some applications to deliver pacing pulses to the atrium or ventricle.

One alternative cardiac pacing technique for treating tachyarrhythmias involves placing a lead in the right ventricle to pace and/or defibrillate the high septal or outflow tract of a heart from the right ventricle. Studies in the literature have demonstrated hemodynamic superiority of right ventricular outflow tract and septal pacing compared with right ventricular apical pacing.

Conventional leads are not designed for septal pacing. Accordingly, there is a need for a lead that is able to perform efficient septal pacing.

US 6, 178, 355 and US 5, 871, 530 each disclose a cardioversion/defibrillation lead comprising an elongated lead body. A centrally disposed lead extension having a smaller diameter than the body and a plurality of elongated, un-insulated wire filaments surrounding the lead extension in a spaced relationship extend from the distal end of the body. A pace/sense helical screw-in electrode adapted to be screwed into the myocardium at a selected site within a heart chamber is disposed at the distal end of the lead extension. The screw-in electrode and the wire filaments are connected to respective conductors disposed within the lead extension and body. The wire filaments form a distributed cardioversion/defibrillation electrode.

### Summary

The present invention provides a lead assembly as claimed in claim 1.

Thus, the lead assembly includes a body with a proximal end and a distal end and at least one conductor disposed within the body. A distal electrode is at the distal end of the body and is electrically coupled with at least one of the conductors. A first electrode is on the body and is electrically coupled with at least one of the conductors. The lead assembly further includes a septum-contacting drop down neck that is less stiff than the body. The neck is between the distal end of the body and the first electrode assembly. A fixation mechanism at the distal end of the body is capable of securing the lead assembly to a interventricular septum, or outflow tract, in a heart.

The lead assembly can be used to supply septal therapy to a patient that is suffering some heart ailment, such as a tachyarrhythmia. In some embodiments, a catheter can be used to insert the lead assembly into the right ventricle of a heart. Once the distal end of the lead assembly is attached to an upper portion of the septum, the catheter is removed.

The relatively low stiffness of the neck allows the neck to drop down along the septum or right ventricle such that any electrodes on the neck and body of the lead assembly make good conforming contact with the septum and/or right ventricle. The benefits of better contact between electrodes that provide therapy to the heart and the tissue of the heart are known to those of ordinary skill in the art. The neck may have a length between about 10mm and about 30mm.

The lead assembly can be used in a method which includes advancing the lead assembly using a catheter until the lead assembly is near tissue in a heart, such as a septum. The method further includes affixing a distal end of the body to the septum and removing the catheter to allow at least a portion of the neck to drop against the septum.

Since the neck of the lead assembly is less stiff than the body, the neck drops down against a septum in a heart once whatever insertion device that is used to attach the distal end of the lead assembly to the septum is removed from the heart.

Depending on the number, type and location of any electrodes on neck and body of the lead assembly, any number of different types of cardio therapies can be provided to the septum, outflow tract and/or right ventricle (among other areas) of the heart.

These and other embodiments, aspects, advantages, and features of the present invention will be set forth in part in the description which follows, and in part will become apparent to those skilled in the art by reference to the following description of the invention and referenced drawings or by practice of the invention. The aspects, advantages, and features of the invention are realized and attained by means of the instrumentalities, procedures, and combinations particularly pointed out in the appended claims and their equivalents.

### Brief Description of the Drawings

Figure 1 is a plan view illustrating a portion of a lead assembly.
Figure 2 is an enlarged plan view illustrating the tip of the lead assembly shown in Figure 1.
Figure 3 is a plan view illustrating another embodiment of a lead assembly.
Figure 4 is a block diagram illustrating a method in accordance with another embodiment of the invention.
Figure 5 illustrates the lead assembly of Figure 1 inserted into a heart using a catheter.
Figure 6 illustrates the catheter partially removed from the lead assembly of Figure 5.
Figure 7 illustrates the catheter removed from the lead assembly of Figure 5.
Figure 8 is a plan view illustrating another embodiment of a lead assembly.
Figure 9 is an enlarged section view illustrating a portion of the lead assembly of Figure 8.

### Detailed Description

In the following detailed description, reference is made to the accompanying drawings which show by way of illustration specific embodiments in which the invention may be practiced. These embodiments are described in sufficient detail to enable those skilled in the art to practice the invention. It is to be understood that other embodiments may be utilized and that structural changes made such that the following detailed description is not to be taken in a limiting sense.

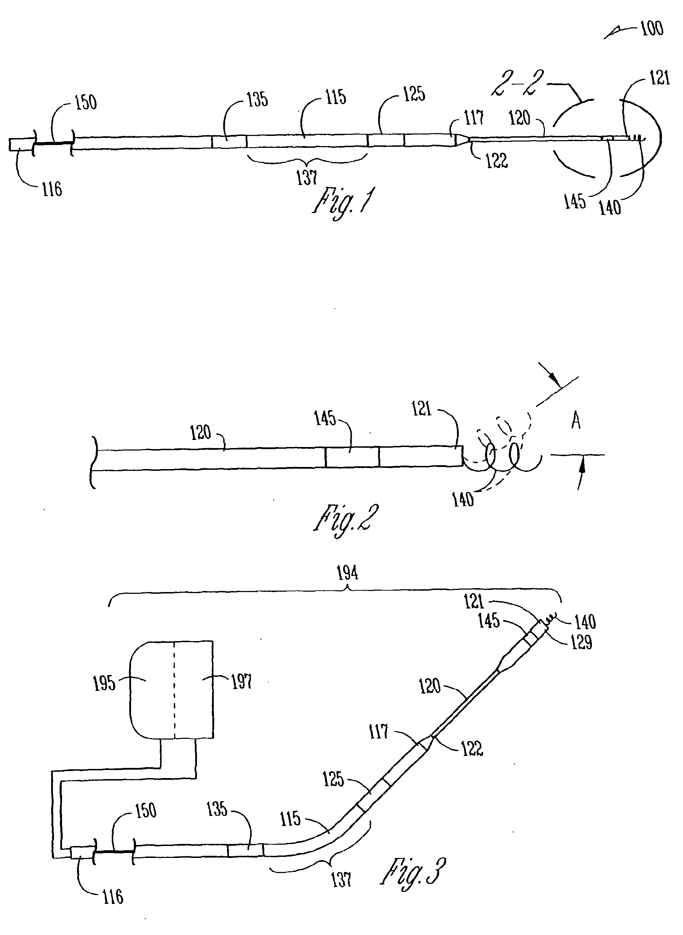

Figure 1 illustrates a lead assembly 100 that includes a body 115 and at least one conductor 150 contained within the body 115. The lead assembly 100 is at least partially formed of a polymer, such as a medical grade silicone rubber for translumenal insertion and access within a living organism such as a patient. The conductor(s) 150 may be any form of conductor, such as, but not limited to, a cable, coil or braided conductor.

The body 115 includes a proximal end 116 and a distal end 121. The lead assembly 100 further includes a neck 120 that is less stiff than the body 115. The neck 120 is between the distal end 121 and a first electrode 125 on the body 115. In some embodiments, the neck 120 has length between about 10mm and about 30mm.

In the illustrated example embodiment, the first electrode 125 is electrically coupled with the at least one conductor 150 at a section 117 adjacent to an end 122 of the neck 120. The first electrode 125 is in the form of a shocking coil, although other types of electrodes may be used.

In some embodiments, the lead assembly 100 includes a second electrode 135 that is also electrically coupled with the at least one conductor 150 and is positioned between the proximal end 116 of the body 115 and the first electrode 125. The second electrode 135 is in the form of a shocking coil, although other types of electrodes may be used.

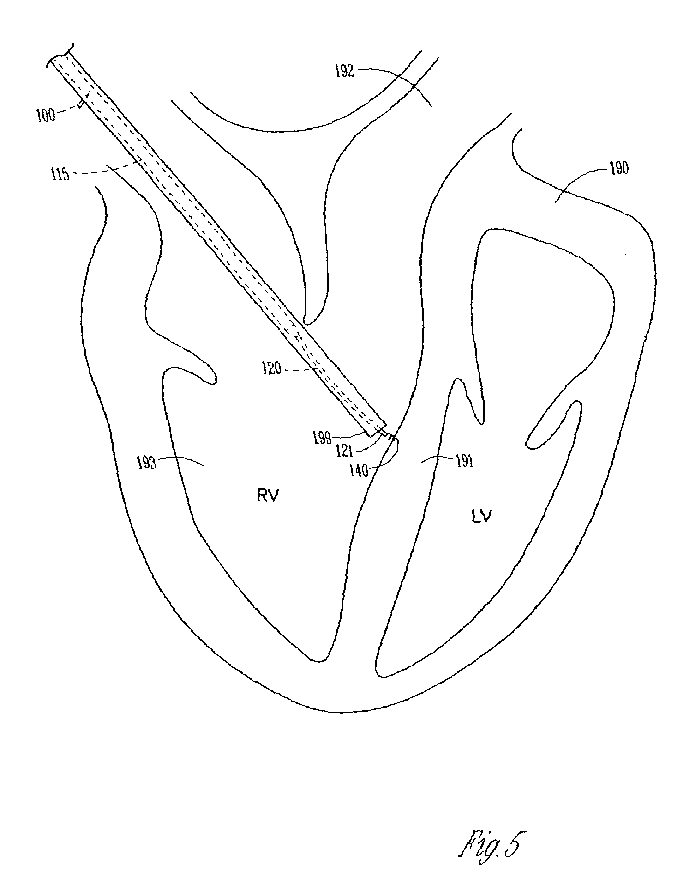

The distal end 121 of the body 115 includes a fixation mechanism, such as a helical coil 140, that is capable of securing the lead assembly 100 to tissue in a heart 190. Although the helical coil 140 is shown in FIG. 1 as being substantially aligned with the longitudinal axis of the assembly 100, the helical coil 140 may be positioned at an angle relative to the longitudinal axis of lead assembly 100 in order to facilitate attachment of the helical coil 140 to tissue of a heart 190, such as the septum 191 and/or the out flow tract 192 of a right ventricle 193 (see also FIGS. 5-7). One example is shown in phantom in Figure 2 where the helical coil 140 is at an angle A relative to the longitudinal axis of the helical coil 140. In some embodiments, angle A is between about 0 and about 90 degrees.

The relatively low stiffness of the neck 120 on the lead assembly 100 allows the lead assembly 100 to be used to apply septal therapy to a patient that is suffering some heart ailment, such as a tachyarrhythmia. The lead assembly 100 is effective in applying such therapy because the neck 120 is adapted to drop against a septum 191 in a heart 190 once a catheter 199 that has been used to insert the lead assembly 100 into the septum 191 is withdrawn from the lead assembly 100.

All, or some, portions of the body 115 and 120 may be coated with an anti-thrombous agent, such as, but not limited to, heparin and polyethxlene glycol. The anti-thrombous agents help to alleviate fibrotic attachment between the lead assembly 100 and heart tissue.

In the illustrated example embodiment, a distal electrode 145 is electrically coupled with the at least one conductor 150 and positioned at the distal end 121 of the neck 120. Distal electrode 145 is in the form of a pacing electrode, although other types of electrodes may be used.

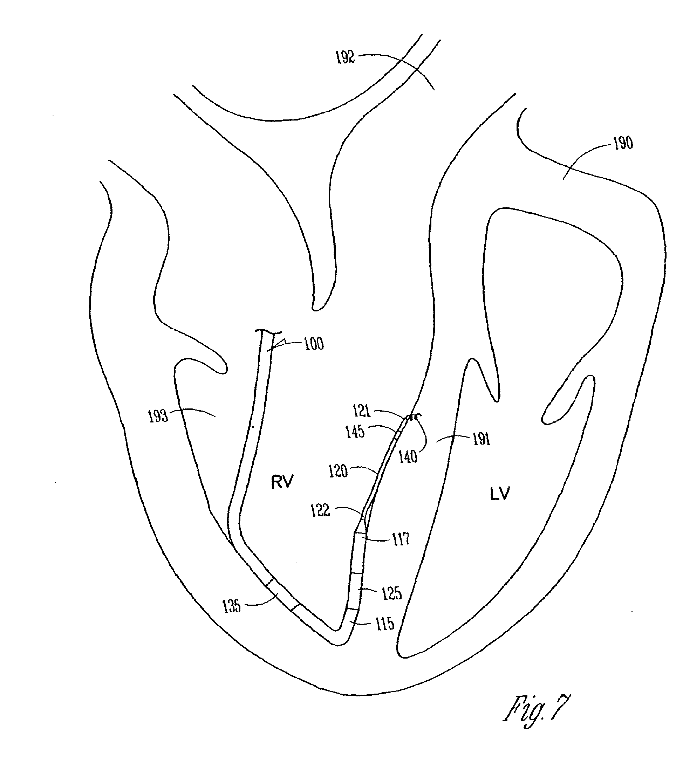

In the embodiment illustrated in Figure 1, a section 137 of the body 115 between the first electrode 125 and the second electrode 135 is straight. In the embodiment shown in Figure 3, section 137 is pre-formed into an arc-shape to facilitate appropriate placement of electrodes assemblies 125, 135 against tissue, such as a septum 191 and/or right ventricle 193 within a heart 190 (see, e.g., FIG. 7).

In the embodiment illustrated in Figure 1, the neck 120 is less stiff than the body 115 because the neck 120 has a cross-sectional area that is smaller than the cross-sectional area of the body 115. In the example embodiment shown in FIG. 3, the distal end 121 of the body 115 includes a section 129 having a cross-sectional area that is larger than the cross-sectional area of the neck 120. In some embodiments, the cross-sectional area of the section 129 is substantially the same as the cross-sectional area of the body 115 (see FIG. 3).

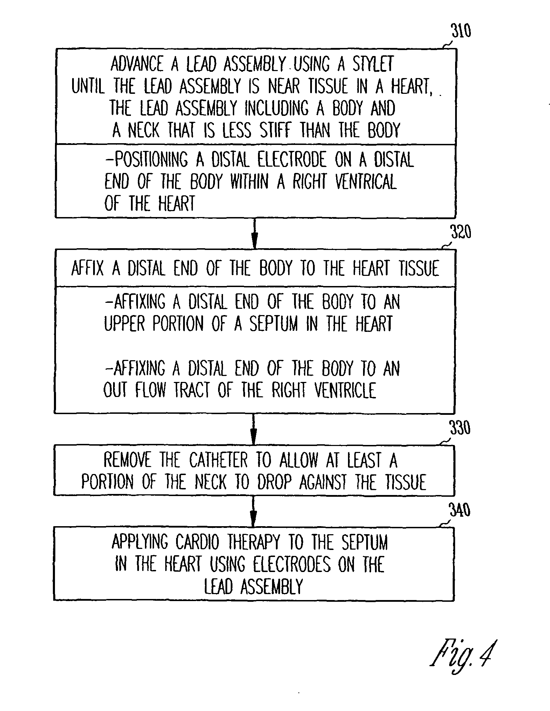

FIG. 4 shows a block diagram illustrating a method 300. The method 300 includes advancing a lead assembly using a catheter until the lead assembly is near tissue in a heart, such as a septum, the lead assembly including a body and a neck that is less stiff than the body 310; affixing a distal end of the body to the heart tissue 320; and removing the catheter to allow at least a portion of the neck to drop against the tissue 330. In some embodiments, the method further includes applying cardio therapy to the septum in the heart using electrodes on the lead assembly 340.

In some embodiments, advancing a lead assembly using a catheter 199 includes positioning the distal end of the body within a right ventricle of the heart. In addition, affixing a distal end of the body to the heart includes affixing a distal end of the body to an upper portion of a septum and/or outflow tract in the heart.

The insertion of lead assembly 100 within heart 190 will be explained further with reference to FIGS. 5-7. A catheter 199 is used to guide the lead assembly 100. The catheter 199 is manipulated to facilitate the insertion of the lead assembly 100 into and through a vein and then through an intracardiac valve. The distal end 121 of the lead body 115 is advanced into the right ventricle 193 of a heart 190 until the distal end 121 is positioned near the septum 191, or outflow tract 192 adjoining the right ventricle 193, of heart 190. In some example embodiments, the distal end of the catheter 199 is curved to facilitate insertion of the lead assembly 100 into an upper portion of the septum 191, or outflow tract 192.

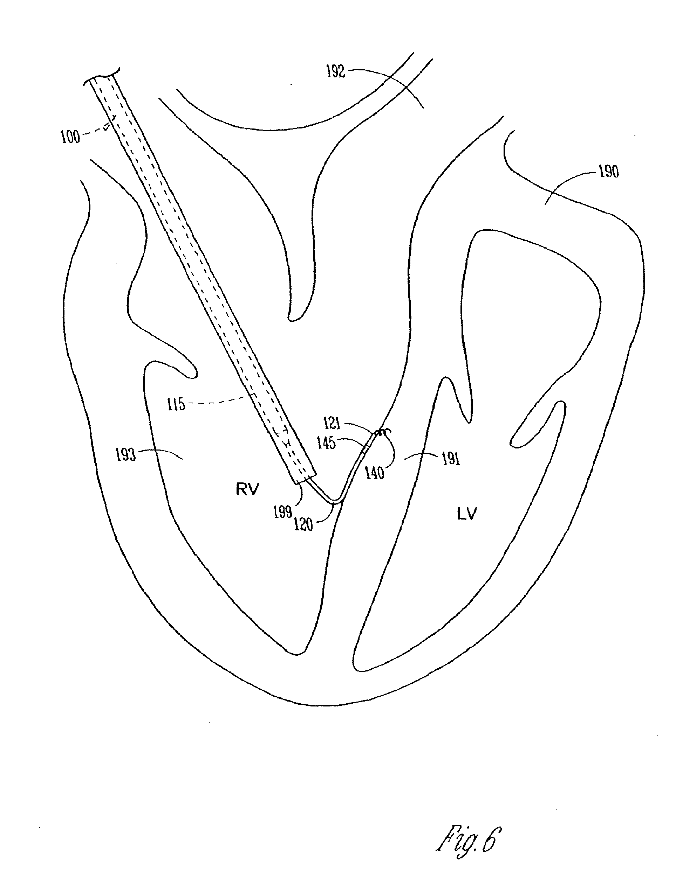

The distal end 121 of the body 115 is affixed to the upper portion of the septum 191 or the out flow tract 192 (see FIG. 5) depending on factors considered by the physician at the time of implantation. As an example, the lead assembly 100 is affixed to the heart 190 by manipulating the catheter 199 to maneuver the helical coil 140 into an upper portion of the septum 191 tissue within the heart 190. Once the distal end 121 of the body 115 is affixed, the catheter 199 is removed such that the exposed portion of the neck 120 begins to drop against the septum 191 (see FIG. 6).

In some embodiments, the first electrode 125 and/or the second electrode 135 drop against the heart tissue (e.g., septum 191 and/or the bottom of right ventricle 193 as shown in FIG. 7). In other embodiments, second electrode 135 is spaced from first electrode 125 such that second electrode 135 is in a supraventricular location. The number and arrangement of electrodes on lead assembly 100 that drop against and engage the septum 191 and/or right ventricle 193 of heart 190 depend on where the distal end 121 of the body 115 engages the heart 190, and the relative locations of any electrodes on the lead assembly 100 relative to the neck 120 and the distal end 121 of the body 115.

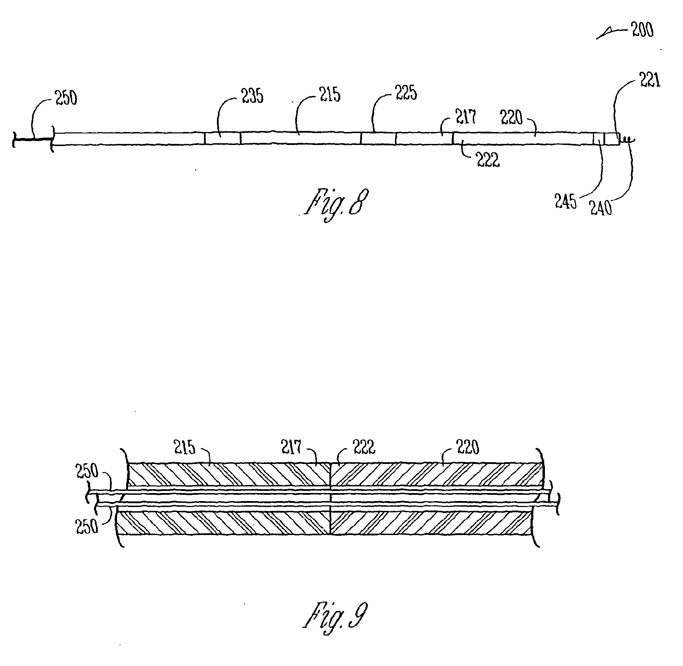

Another lead assembly 200 is shown in FIGS. 8 and 9. The lead assembly 200 similarly includes a body 215 and at least one conductor 250 (two conductors are shown in FIG. 9) contained within the body 215. The body 215 includes a proximal end (not visible in FIGS. 8 and 9) and a distal end 221. A first electrode 225 is electrically coupled with at least one of the conductors 250 and is positioned on the body 215.

The lead assembly 200 further includes a neck 220 that is less stiff than the body 215. The neck 220 is between the distal end 221 of the body 215 and the first electrode 215. The first electrode 225 is positioned on the body 215 at a section 217 that is adjacent to an end 222 of the neck 220.

In some embodiments, the lead assembly 200 includes a second electrode 235 that is also electrically coupled with at least one of the conductors 250, and is positioned between the proximal end of the body 215 and the first electrode 225.

The distal end 221 of the body 215 includes a fixation mechanism, such as a helical coil 240, that is capable of securing the lead assembly 200 to tissue in a heart 190, such as a septum 191. A distal electrode 245 is electrically coupled to at least one of the conductors 250 and is positioned at the distal end 221 of body 215.

In the example embodiment illustrated in FIGS. 8 and 9, the neck 220 is similarly less stiff than the body 215 because the neck 220 is made from a first material that is less stiff than a second material that forms the body 215. One example of the first material that forms the neck 220 is silicone, polyurethane or other biocompatible polymers. In addition, one example of the second material that forms the body 215 is silicone, polyurethane or other biocompatible polymers. In any embodiment, the neck and body of the lead assembly may be integral with one another or fabricated separately and joined together as long as the neck is less stiff than the body.

It should be noted that the lead assembly is meant to encompass all embodiments of a lead assembly that includes a neck and a body where the neck is less stiff than the body. Since the neck of the lead assembly less stiff than the body, the neck can drop against the septum when an insertion device that is used to attach the lead assembly to the septum is removed from the heart. The leads shown and described herein are able to provide any number of different types of cardio therapies to a septum, outflow tract and/or right ventricle (among other areas) of a heart.

The lead assembly 100 may also be part of a system 194 for delivering electrical pulses to stimulate a heart and/or for receiving electrical pulses to monitor the heart. The system 194 (see FIG. 3) includes a pulse generator 195 and/or signal sensor 197. As discussed above, the distal end 121 is adapted for implantation within the heart of a patient and the proximal end 116 is connected directly or indirectly to a terminal connector that electrically connects the various electrodes and conductors within the lead body to the pulse generator 195 and/or signal sensor 197. The pulse generator 195 and/or signal sensor 197 may be implanted pectorally, abdominally, or elsewhere within a patient.

It is to be understood that the above description is intended to be illustrative, and not restrictive. For instance, the leads described above include, but are not limited to, tachy or brady leads. It should be noted that features of the various above-described embodiments may be interchanged to form additional combinations. Many other embodiments will be apparent to those of skill in the art upon reviewing the above description. The scope of the invention should be determined with reference to the appended claims.

## Claims

1. A lead assembly (100, 200) for stimulating a heart (190) from one or both of an outflow tract (192) or an interventricular septum (191) comprising:
a body (115, 215) including a proximal end (116) and a distal end (121, 221);
at least one conductor (150, 250) disposed within the body;
a distal electrode (145, 245) at the distal end of the body, the distal electrode electrically coupled with at least one of the conductors;
a first electrode (125, 225) on the body, the first electrode electrically coupled with at least one of the conductors;
a septum-contacting drop down neck (120, 220) between the distal end of the body and the first electrode, the neck being less stiff than the body thereby allowing the neck to drop down along the septum or right ventricle such that any electrodes on the neck and body of the lead assembly make good conforming contact with the septum and/or right ventricle; and
a fixation mechanism at the distal end of the body, the fixation mechanism capable of securing the distal end to the outflow tract or the septum.

2. The lead assembly of claim 1, wherein the septum-contacting drop down neck drops against the septum such that the distal electrode and the first electrode engage the septum.

3. The lead assembly of any of claims 1 or 2, further comprising a second electrode (135, 235) coupled with at least one of the conductors, the second electrode positioned between the proximal end of the body and the first electrode.

4. The lead assembly of claim 3, wherein the septum-contacting drop down neck drops against the septum such that the second electrode engages a bottom portion of a right ventricle (193).

5. The lead assembly of claim 3, wherein a section (137) of the body between the first electrode and the second electrode is arc-shaped.

6. The lead assembly of any of claims 1-5, wherein the fixation mechanism is a helical coil (140, 240).

7. The lead assembly of claim 6, wherein the helical coil is aligned with a longitudinal axis of the body.

8. The lead assembly of claim 6, wherein the helical coil is at an angle (A) between about 0 and 90 degrees relative to a longitudinal axis of the body.

9. The lead assembly of any of claims 1-8, wherein the septum-contacting drop down neck has a length between about 10mm and about 30 mm.

10. The lead assembly of any of claims 1-9, wherein the body has a first cross-sectional area and the septum-contacting drop down neck has a second cross-sectional area that is smaller than the first cross-sectional area of the body.

11. The lead assembly of claim 10, wherein the distal end of the septum-contacting drop down neck includes a section (129) that has a third cross-sectional area which is different than the second cross-sectional area.

12. The lead assembly of claim 11, wherein the third cross-sectional area of the section at the distal end of the septum-contacting drop down neck is substantially the same size as the first cross-sectional area of the body.

13. The lead assembly of any of claims 1-12, wherein the body is made of a first material and the neck is made of a second material.

14. The lead assembly of claim 13, wherein the first material is silicone or polyurethane.

15. The lead assembly of any of claims 1-14, wherein the body is coated with an anti-thrombus agent.

16. The lead assembly of claim 15, wherein the anti-thrombus agent is one or a combination of heparin or polyethxlene glycol.

17. The lead assembly of any of claims 1-16, wherein the first electrode is a shocking coil.

18. The lead assembly of any of claims 1-17, wherein the distal electrode is a sensing electrode.

19. The lead assembly of any of claims 1-18, further comprising a pulse generator (195) electrically coupled to at least one of the conductors.

20. The lead assembly of any of claims 1-19, further comprising a signal sensor (197) electrically coupled to at least one of the conductors.

21. The lead assembly of any of claims 1-20, wherein the septum-contacting drop down neck drops against one or both of the outflow tract or the septum upon the removal of a guide catheter (199).

## Patentansprüche

1. Leitungsanordnung (100, 200) zur Stimulation eines Herzens (190) von einem Abflußweg (192) oder einem Kammerseptums (191) oder von beiden aus, umfassend:
einen Körper (115, 215) mit einem proximalen Ende (116) und einem distalen Ende (121, 221),
mindestens eine innerhalb des Körpers angeordnete Leitung (150, 250),
eine distale Elektrode (145, 245) am distalen Ende des Körpers, wobei die distale Elektrode elektrisch mit mindestens einer der Leitungen verbunden ist,
eine erste Elektrode (125, 225) auf dem Körper, wobei die erste Elektrode mit mindestens einer der Leitungen verbunden ist,
einen septumberührenden herabsinkenden Hals (120, 220) zwischen dem distalen Ende des Körpers und der ersten Elektrode, wobei der Hals weniger steif als der Körper ist und es **dadurch** dem Hals erlaubt, entlang des Septums oder des rechten Ventrikels derart herabzusinken, daß beliebige Elektroden auf dem Hals und dem Körper der Leitungsanordnung einen gut angepaßten Kontakt mit dem Septum und/oder rechten Ventrikel herstellen, und
einen Fixationsmechanismus am distalen Ende des Körpers, wobei der Fixationsmechanismus in der Lage ist, das distale Ende am Abflußweg oder am Septum zu sichern.

2. Leitungsanordnung nach Anspruch 1, wobei der septumberührende herabsinkende Hals gegen das Septum herabsinkt, so daß die distale Elektrode und die erste Elektrode das Septum berühren.

3. Leitungsanordnung nach einem der Ansprüche 1 oder 2, ferner umfassend eine zweite Elektrode (15, 235), die mit mindestens einer der Leitungen gekoppelt ist, wobei die zweite Elektrode zwischen dem proximalen Ende des Körpers und der ersten Elektrode angeordnet ist.

4. Leitungsanordnung nach Anspruch 3, wobei der septumberührende herabsinkende Hals gegen das Septum herabsinkt, so daß die zweite Elektrode einen unteren Teil eines rechten Ventrikels (193) berührt.

5. Leitungsanordnung nach Anspruch 3, wobei ein Abschnitt (137) des Körpers zwischen der ersten Elektrode und der zweiten Elektrode bogenförmig ausgebildet ist.

6. Leitungsanordnung nach einem der Ansprüche 1 bis 5, wobei der Fixationsmechanismus eine schraubenförmige Spule (140, 240) ist.

7. Leitungsanordnung nach Anspruch 6, wobei die schraubenförmige Spule mit der Längsachse des Körpers ausgerichtet ist.

8. Leitungsanordnung nach Anspruch 6, wobei die schraubenförmige Spule in einem Winkel (A) zwischen ungefähr 0 und 90 Grad relativ zur Längsachse des Körpers steht.

9. Leitungsanordnung nach einem der Ansprüche 1 bis 8, wobei der septumberührende herabsinkende Hals eine Länge zwischen ungefähr 10 mm und ungefähr 30 mm aufweist.

10. Leitungsanordnung nach einem der Ansprüche 1 bis 9, wobei der Körper eine erste Querschnittsfläche hat und der septumberührende herabsinkende Hals eine zweite Querschnittsfläche hat, die kleiner ist als die erste Querschnittsfläche des Körpers.

11. Leitungsanordnung nach Anspruch 10, wobei das distale Ende des septumberührenden herabsinkenden Halses einen Abschnitt (129) aufweist, der eine dritte Querschnittsfläche besitzt, die sich von der zweiten Querschnittsfläche unterscheidet.

12. Leitungsanordnung nach Anspruch 11, wobei die dritte Querschnittsfläche des Abschnitts am distalen Ende des septumberührenden herabsinkenden Halses im wesentlichen die gleiche Größe wie die Querschnittsfläche des Körpers besitzt.

13. Leitungsanordnung nach einem der Ansprüche 1 bis 12, wobei der Körper aus einem ersten Material besteht und der Hals aus einem zweiten Material besteht.

14. Leitungsanordnung nach Anspruch 13, wobei das erste Material Silikon oder Polyurethan ist.

15. Leitungsanordnung nach einem der Ansprüche 1 bis 14, wobei der Körper mit einem anti-thrombotischen Stoff beschichtet ist.

16. Leitungsanordnung nach Anspruch 15, wobei der anti-thrombotische Stoff Heparin oder Polyethylen-Glykol oder eine Kombination davon ist.

17. Leitungsanordnung nach einem der Ansprüche 1 bis 16, wobei die erste Elektrode eine Schockspule ist.

18. Leitungsanordnung nach einem der Ansprüche 1 bis 17, wobei die distale Elektrode eine Meßelektrode ist.

19. Leitungsanordnung nach einem der Ansprüche 1 bis 18, ferner umfassend einen Impulsgenerator (195), der elektrisch mit mindestens einer der Leitungen verbunden ist.

20. Leitungsanordnung nach einem der Ansprüche 1-19 ferner umfassend einen Signalsensor (197), der elektrisch mit mindestens einer der Leitungen verbunden ist.

21. Leitungsanordnung nach einem der Ansprüche 1-20, wobei der septumberührende herabsinkende Hals nach Entfernung eines Leitkatheters (199) gegen den Abflußweg oder das Septum oder beide herabsinkt.

## Revendications

1. Câblage (100, 200) pour stimuler un coeur (190) de l'un d'un tractus d'évacuation (192) ou d'un septum interventriculaire (191) ou de ces deux éléments, comprenant :
un corps (115, 215) comprenant une extrémité proximale (116) et une extrémité distale (121, 221) ;
au moins un conducteur (150, 250) disposé à l'intérieur du corps ;
une électrode distale (145, 245) au niveau de l'extrémité distale du corps, l'électrode distale étant électriquement couplée à au moins un des conducteurs ;
une première électrode (125, 225) sur le corps, la première électrode étant électriquement couplée à au moins un des conducteurs,
un goulot rabattable en contact avec le septum (120, 220) entre l'extrémité distale du corps et la première électrode, le goulot étant moins rigide que le corps, permettant ainsi au goulot d'être rabattu le long du septum ou du ventricule droit de sorte que les électrodes sur le goulot et le corps du câblage se trouvent en bon contact en épousant le septum et/ou le ventricule droit ; et
un mécanisme de fixation au niveau de l'extrémité distale du corps, le mécanisme de fixation étant capable de fixer l'extrémité distale au tractus d'évacuation ou au septum.

2. Câblage selon la revendication 1, dans lequel le goulot rabattable en contact avec le septum se rabat contre le septum de sorte que l'électrode distale et la première électrode viennent en prise avec le septum.

3. Câblage selon l'une quelconque des revendications 1 ou 2, comprenant en outre une seconde électrode (135, 235) couplée avec au moins un des conducteurs, la seconde électrode étant positionnée entre l'extrémité proximale du corps et la première électrode.

4. Câblage selon la revendication 3, dans lequel le goulot rabattable en contact avec le septum se rabat contre le septum de sorte que la seconde électrode vient en prise avec une partie inférieure d'un ventricule droit (193).

5. Câblage selon la revendication 3, dans lequel une section (137) du corps entre la première électrode et la seconde électrode est en forme d'arc.

6. Câblage selon l'une quelconque des revendications 1 à 5, dans lequel le mécanisme de fixation est une bobine hélicoïdale (140, 240).

7. Câblage selon la revendication 6, dans lequel la bobine hélicoïdal est alignée avec un axe longitudinal du corps.

8. Câblage selon la revendication 6, dans lequel la bobine hélicoïdale se trouve à un angle (A) compris entre environ 0 et 90 degrés par rapport à un axe longitudinal du corps.

9. Câblage selon l'une quelconque des revendications 1 à 8, dans lequel le goulot rabattable en contact avec le septum a une longueur comprise entre environ 10 mm et environ 30 mm.

10. Câblage selon l'une quelconque des revendications 1 à 9, dans lequel le corps a une première aire en coupe et le goulot rabattable en contact avec le septum a une deuxième aire en coupe qui est plus petite que la première aire en coupe du corps.

11. Câblage selon la revendication 10, dans lequel l'extrémité distale du goulot rabattable en contact avec le septum comprend une section (129) qui présente une troisième aire en coupe qui est différente de la deuxième aire en coupe.

12. Câblage selon la revendication 11, dans lequel la troisième aire en coupe de la section au niveau de l'extrémité distale du goulot rabattable en contact avec le septum a des dimensions sensiblement identiques à celle de la première aire en coupe du corps.

13. Câblage selon l'une quelconque des revendications 1 à 12, dans lequel le corps est constitué d'un premier matériau et le goulot est constitué d'un second matériau.

14. Câblage selon la revendication 13, dans lequel le premier matériau est en silicone ou en poly(uréthane).

15. Câblage selon l'une quelconque des revendications 1 à 14, dans lequel le corps est revêtu d'un agent anti-thrombus.

16. Câblage selon la revendication 15, dans lequel l'agent anti-thrombus est un élément parmi l'héparine ou le poly(éthylène glycol), ou une combinaison de ceux-ci.

17. Câblage selon l'une quelconque des revendications 1 à 16, dans lequel la première électrode est une bobine de choc.

18. Câblage selon l'une quelconque des revendications 1 à 17, dans lequel l'électrode distale est une électrode de détection.

19. Câblage selon l'une quelconque des revendications 1 à 18, comprenant en outre un générateur d'impulsions (195) couplé électriquement à au moins un des conducteurs.

20. Câblage selon l'une quelconque des revendications 1 à 19, comprenant en outre un détecteur de signaux (197) couplé électriquement à au moins un des conducteurs.

21. Câblage selon l'une quelconque des revendications 1 à 20, dans lequel le goulot rabattable en contact avec le septum se rabat contre l'un du tractus d'évacuation ou du septum ou les deux au moment du retrait d'un cathéter guide (199).
